# EUROPEAN PATENT SPECIFICATION

(11) **EP 4 069 342 B1**
(45) Date of publication and mention of the grant of the patent: **07.10.2026**
(21) Application number: 20820582.3
(22) Date of filing: 02.12.2020
(51) Int. Cl.: A61M 13/00, A61B 17/34

(54) **LOW SOURCE IMPEDANCE INSUFFLATOR**
INSUFFLATIONSGERÄT MIT NIEDRIGER QUELLENIMPEDANZ
INSUFFLATEUR À FAIBLE IMPÉDANCE DE SOURCE

(30) Priority: 02.12.2019 NL 2024352
(43) Date of publication of application: 12.10.2022
(73) Proprietor: Spatium Medical B.V., 3015 GD Rotterdam (NL)
(72) Inventor: STERKE, Frank, 3015 GE Rotterdam (NL); VAN WETERINGEN, Willem, 3015 GE Rotterdam (NL); DELLACÀ, Raffaele Lorenzo, 20133 Milano (IT)
(74) Representative: V.O.
(86) International application number: PCT/NL2020/050752
(87) International publication number: WO 2021/112672

(56) References cited:
- WO-A1-2005/035035
- WO-A1-2017/014623
- US-B2- 7 431 031

## Description

### FIELD

The present invention relates to an insufflation apparatus intended to expose structures within a cavity of the human body, by insufflating gas into that body cavity, to obtain a field of vision, through the endoscope, to perform a diagnostic and/or therapeutic endoscopic procedure to that structure in the body cavity. The apparatus further relates to a computer-controlled method of operating an insufflator intended to expose structures within a cavity of the human body for a diagnostic and/or therapeutic endoscopic procedure.

### BACKGROUND

The application of minimal access surgery, allowing surgery through a few small incisions by introduction of a camera and instruments, has become the standard for most surgical procedures. In the thorax and abdomen a surgical field is created by the insufflation of e.g. carbon dioxide gas to the desired pressure level.

Gas insufflation for minimal access surgery was developed in the 1950's. First oxygen, and the carbon dioxide gas was insufflated under pressure into the abdominal cavity in order to create surgical workspace. Insufflation devices apply a set pressure and gas flow in order to create and maintain the required gas volume. This concept has remained unchanged to date in clinical practice. Insufflators have very limited adaptation abilities to cope with pressure changes originating from the patient. As a consequence the insufflated gas volume is relatively constant. It is known that the pressure that is applied to the body cavity influences the balance of pressures within the body. For example the pressure required for ventilating the lungs of a patient during a surgical procedure is opposed by the pressure created by the insufflation device. Another well-known effect is on haemodynamics, caused by the fact that venous blood pressures are typically lower than commonly used insufflation pressures. As a consequence venous return is impaired, affecting systemic circulation.

Current insufflators use high-pressure insufflator gas sources and inject the gas into the cavity by controlling the inflow by a valve. As these pneumatic systems have a very high impedance for the outflow gas (i.e. they do not permit return flow), all insufflators need a release valve that opens in the case the pressure inside the cavity increases over the pre-set one. Because of this arrangement, rapid pressure variations occurring in the cavity due to, for example, mechanical ventilation, coughing or similar, cannot be dynamically compensated and, therefore, large rapid pressure variations are commonly observed in the cavity. US7431031 describes an insufflation system having a vent assembly.

### SUMMARY

In an aspect of the invention there is provided an insufflator apparatus for exposing structures within a cavity of the human body for a diagnostic and/or therapeutic endoscopic procedure, comprising: an insufflation gas supply, adapted to provide insufflation gas to a pressure regulator; the pressure regulator, adapted to supply insufflation gas into the cavity of the human body via an input mechanism attachable to the human body, a means for determining a pressure level in the body cavity; an insufflator vent mechanism adapted to release excess insufflation gas volume returning from the pressure regulator; an insufflator controller arranged to real time adapt an insufflation rate of said insufflator gas via said gas supply and vent mechanism at a set average pressure level in the body cavity in accordance with the means for determining the pressure level in the body cavity; and wherein the pressure regulator has a limited volume for temporarily storing a gas returning from the body cavity to thereby avoid transient pressure deviations from the set average pressure level in the body cavity, e.g. due to coughing or mechanical ventilation and allowing the gas to return to the body cavity to maintain the set average pressure.
The invention allows to keep insufflation pressure highly constant within the body cavity by allowing flow moving in and out of the surgical workspace (the 'body cavity') at a low impedance effectively provided by the pressure regulator. This allows accommodating for any rapid change in body cavity pressure as a consequence not only to the tidal changes of ventilation pressures but also to coughing or any other reason. The added value of this is a reduced burden of insufflation on patients' ventilation and haemodynamics without the need of the insufflator to be synchronised to the mechanical ventilator. This invention leads to a stand-alone insufflation device able to implement a fast compensation of surgical space pressure perturbation without requiring interfacing to other devices or connections to breathing circuits. Moreover, the presence of a gas reservoir allows maintaining a stable pressure in the body cavity while minimizing the venting of insufflation gas.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figure 1A schematically depicts a first embodiment according to an aspect of the invention having a turbine-based setup connected to a high compliance reservoir;
Figure 1B schematically depicts a second embodiment according to an aspect of the invention;
Figure 2A schematically depicts a third embodiment according to an aspect of the invention;
Figure 2B schematically depicts a fourth embodiment according to an aspect of the invention;
Figure 3A schematically depicts a fifth embodiment according to an aspect of the invention;
Figure 3B schematically depicts a sixth embodiment according to an aspect of the invention;
Figure 4 (A and B) schematically depicts a seventh embodiment according to an aspect of the invention;
Figure 5 schematically depicts an embodiment of an insufflator, in conjunction with a breathing controller;
Figure 6A shows an illustrative graph of a set pressure level having the inventive characteristic, in comparison with a standard insufflator
Figure 6B shows an illustrative graph of breathing pressure trace compared to a standard insufflator.

### DETAILED DESCRIPTION

Unless otherwise defined, all terms (including technical and scientific terms) used herein have the same meaning as commonly understood by one of ordinary skill in the art to which this disclosure belongs as read in the context of the description and drawings. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some instances, detailed descriptions of well-known devices and methods may be omitted so as not to obscure the description of the present systems and methods. Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be further understood that the terms "comprises" and/or "comprising" specify the presence of stated features but do not preclude the presence or addition of one or more other features. All publications, patent applications, patents, and other references mentioned herein are incorporated by reference in their entirety. In case of conflict, the present specification, including definitions, will control.
The term "insufflator" is used to denote a device for exposing by insufflation of pressurized gas structures within a cavity of the human body for a diagnostic and/or therapeutic endoscopic procedure. Exemplary body cavities may be the thoracic or abdominal cavity. By insufflation, it is meant to insufflate an insufflator gas, most commonly CO₂ at controlled gas flow, gas output volume and/or gas output pressure in particular. The instantaneous pressure may be measured at sample rates of 0.1-500 Hz or even higher sample rates at least with a wave recognition up to 20 Hz in order to suitably predict and control the dynamics of the insufflated gas and dynamic response of the human body and body wall during gas insufflation. In the examples, insufflation may also encompass exsufflation, i.e. active removal of (part of) the insufflated volume.

The term 'insufflation rate' may denote a physical parameter, such as pressure, volume, temperature and frequency, that is adjusted in accordance with an instantaneous inflated lung volume, by means of hard-wired coupling with the ventilator or high-frequency internal or external pressure and/or flow sensors.

Both the insufflator gas and the ventilation gas may be conditioned, e.g. humidified by using a humidifier or brought to a certain temperature by a heating installation. The term 'real time' is indicated to substantially continuously measure and control, in contrast to isolated control that has a sample frequency larger than a breathing frequency. Typically, for real time measurement and control, a sample frequency of at least twice the breathing frequency is desirable.

The "insufflator controller" may be a dedicated processor for performing in accordance with the present system or may be a general-purpose processor wherein only one of many functions operates for performing in accordance with the present system. The processor may operate utilizing a program portion, multiple program segments, or may be a hardware device utilizing a dedicated or multi-purpose integrated circuit. Any type of processor may be used such as dedicated or shared one. The processor may include micro-controllers, central processing units (CPUs), digital signal processors (DSPs), ASICs, or any other processor(s) or controller(s) such as digital optical devices, or analog electrical circuits that perform the same functions, and employ electronic techniques and architecture. The controller or processor may further comprise a memory that may be part of or operationally coupled to the controller. The memory may be any suitable type of memory where data is stored. Any medium known or developed that can store and/or transmit information suitable for use with the present systems and methods may be used as a memory. The memory may also store user preferences and/or application data accessible by the controller for configuring it to perform operational acts in accordance with the present systems and methods.

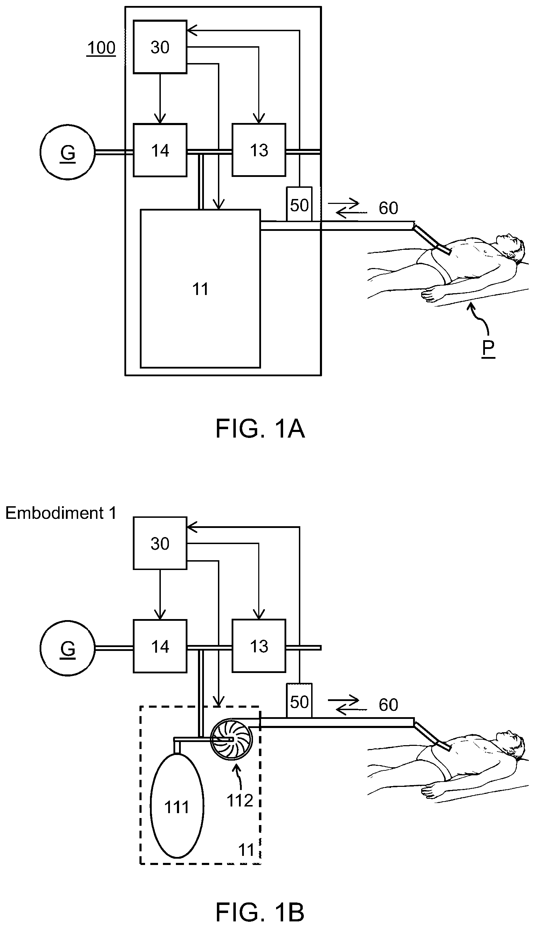

Figure 1A schematically shows an embodiment of the invention, wherein an insufflator apparatus 100 is provided for exposing structures within a cavity of the human body for diagnostic and/or therapeutic endoscopic procedure. In the figure this is schematically indicated by the practical use of the device 100, when operated to supply gas to a patient P, in particular, into the cavity of the body via an input channel 60. At least one sensor 60 is arranged for measuring a pressure level in the body cavity as a means for determining the pressure level in the body cavity. The device 100 comprises an insufflator vent mechanism 13, e.g. in the form of a deflation valve, adapted to control thepressure regulator 11 to a set pressure level in the input channel 60, in particular when fluidly connected to the body cavity. An insufflator controller 30 is arranged to real time adapt an insufflation rate, e.g. via a supply valve 14, vent mechanism 13 and the pressure regulator 11 at a set average pressure level in the body cavity in accordance with a sensed pressure of the at least one sensor 50. The supply valve 14 is connected to an external gas source G. The pressure regulator 11 is comprising a compliance mechanism in the form of a limited volume for temporarily storing a gas returning from the body cavity to thereby avoid transient pressure deviations from the set average pressure level in the body cavity, e.g. due to coughing or mechanical ventilation and allowing the gas to return to the body cavity to maintain the set average pressure.
The disclosed system is intended for exposing an intended structure within a cavity of the human body for therapeutic and/or surgical treatment, using insufflator 100. The insufflator comprising an insufflator input mechanism 60 adapted to input the gas from the gas supply G into a cavity of a human body. In the gas supply G a gas container may be present, wherein a suitable insufflation gas is stored, or the gas can be supplied via an external gas supply, e.g. a wall socket.

The pressure regulator 11 comprises a gas reservoir which may provide in- and exsufflation by mechanically changing its volume for temporarily storing a gas returning from the body cavity to avoid transient pressure deviations from the set average pressur level in the body cavity. An insufflator controller 30 is provided for setting and maintaining a pre-defined gas pressure in the cavity by compensating the fast transient pressure changes, firstly with the pressure regulator 11 and secondly with the supply valve 14 and vent mechanism 13. The insufflator controller 30 is provided for enlarging the cavity by the insufflation of gas from the gas supply G into the cavity.

The described invention is implementing a two-way low impedance gas pressure supply, which inherently permits easy gas flow in and out of the cavity, regardless of the origin of the pressure change that causes the gas flow, The insufflation system 100 may compensate for transient pressure changes in the cavity due to breathing, being totally independent, e.g. from the mechanical ventilator. Therefore, it does not need any means to synchronize to it and, consequently, can be used in combination of all existing and future mechanical ventilators. In principle, the low impedance gas supply may be passive, but it is advantageous when the compliance mechanism is controlled by the insufflator controller as a function of the measured pressure variations in the body cavity since then, faster response times and low pressure peaks can be attained by actively control of the compliance mechanism, e.g. under control of the insufflator controller 30. The compliance mechanism has a limited volume, of e.g. 250 milliliters up to more than 5 liters, for storing, i.e. buffering and enabling return of gas flow from and to the body cavity. This provides a convenient way of countering pressure variations, while at the same time preventing that insufflator gas is released by the vent mechanism. A limited volume may be a non-fixed volume that varies between a minimum and maximum volume.

Figure 1B schematically depicts a first embodiment (1) of an insufflator according to an aspect of the invention having a turbine-based setup connected to a high compliance reservoir. In particular, the pressure regulator 11 is comprised of an elastic compliance bag 111, communicatively coupled to the input channel 60. A typical compliance bag may be a so-called Douglas bag, known for lung ventilation purposes, as a high compliance reservoir. In addition a fan 112 (compressor) is used to increase the pressure of the insufflation gas in the reservoir to the set pressure level of the cavity. In this embodiment the low impedance insufflator is obtained by connecting the pressurised gas supply valve 14 to a high-compliance reservoir 111 (such as a breathing bag). This bag 111 is kept partially filled with insufflation gas by controlling the supply valve 14 and vent mechanism 13. The reservoir is then connected to the inlet of a fan 112 of which the motor is controlled by a microcontroller system 30 to keep the insufflation pressure constant. As the turbine 112 constitutes a low-impedance pressure generator, in case of increases of the cavity pressure the gas in the body will automatically flow out back to the reservoir without requiring the operation of an exhalation valve 13. The advantage of using a fan is that it has a low internal resistance, allowing return flow resulting from transient pressure variations due to e.g. coughing or breathing pressure variations or other unexpected pressure changes. In combination with a compliance bag, that functions as a capacitance for temporary storage of return gas flow, this functions as an excellent impedance reduction system to accommodate pressure variations in the body cavity even without a need for real-time servo control of the fan.

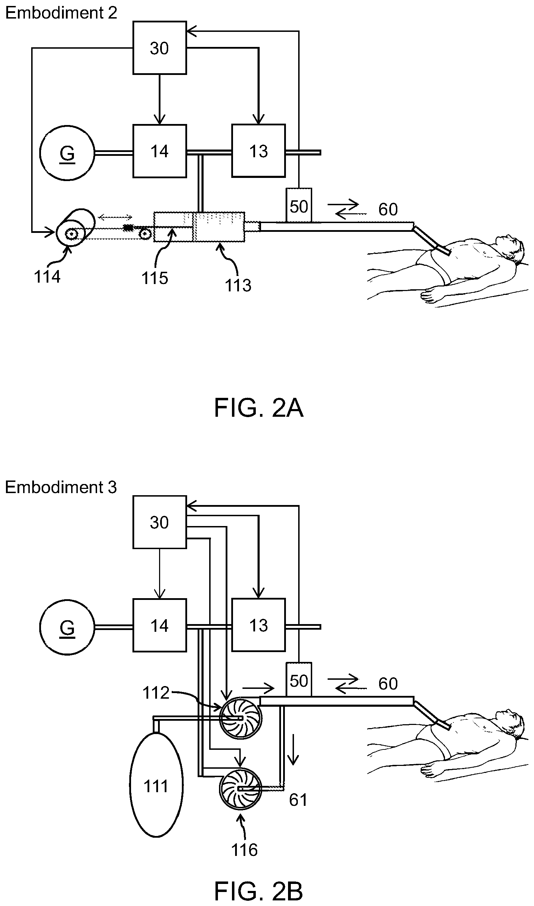

Figure 2A shows another embodiment having a pressure regulator that is controlled by the insufflator controller 30 as a function of the measured pressure variations in the body cavity. The use of a closed-loop control system 30 for controlling the insufflator may change a high impedance pressure generator into behaving as a low-impedance one. In the example, specifically, the compliance mechanism comprises a piston controlled cylinder 113 communicatively coupled to the input channel 60, wherein the piston is actively controlled by the insufflator controller 30 to counteract cavity pressure variations. Thus, by actuating the piston 115 under control of an actuator 114, in correspondence with the pressure sensing means in the body cavity small volumes of gas, e.g. 250 milliliters up to more than 5 liters, corresponding to pressure variations, e.g. due to coughing or breathing, can be accomodated, while keeping the pressure in the body cavity substantially constant. A possible embodiment is made by connecting the pressurised gas source to a motor-activated piston-cylinder system. The cylindrical chamber is then connected to the body cavity. The piston position may be servo-controlled by a closed loop system for maintaining the pressure in the cavity constant, allowing the gas in the cavity flowing out of the body and filling the cylinder in case of rapid increases of the pressure. A supply valve 14 can be used to refill the cylinder 113, the vent 13 mechanism can be used to empty the cylinder 113. In these phases the pressure in the system is kept constant by the closed loop piston controller.

Figure 2B shows a further enhancement of Embodiment 1, where the pressure regulator 11 is comprised of an elastic compliance bag 111, that is communicatively coupled to the input channel 60, in cooperation with a fan 112 (compressor) that is in the input channel 60 controlled by the insufflator controller to provide a controllable pressure difference. In addition to the Embodiment 1, Embodiment 3 foresees that the return gas flow is provided via an output channel 61 for providing a return gas flow via a channel at least partially distinct from the input channel 60. The compliance bag 111 is communicatively coupled with two parallel turbines 112, 116 for insufflation and for exsufflation respectively. The output channel 61 and the input channel 60 originate from a single orifice connected to the body cavity thus providing a single connection to the body cavity access.

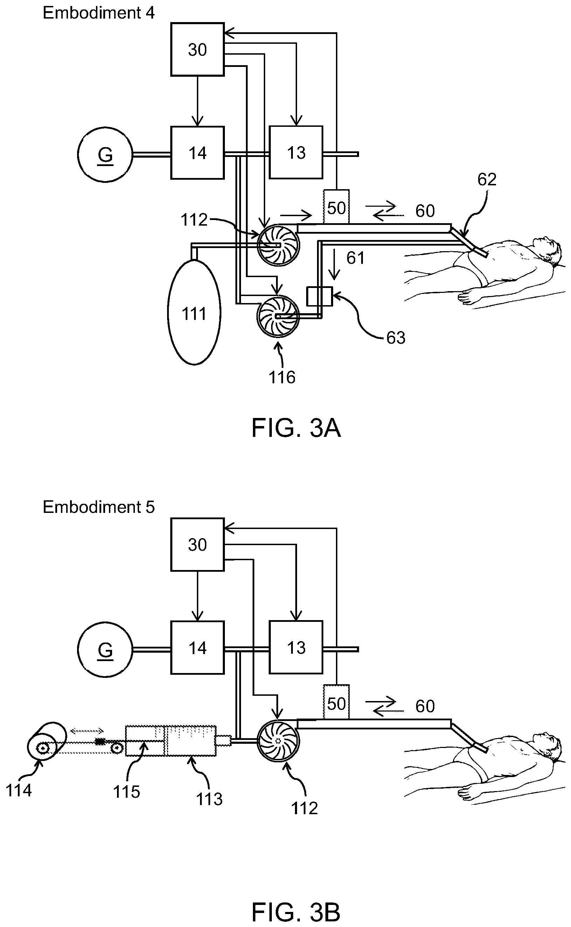

Embodiment 4, shown in Figure 3A shares some features with Embodiment 3. In addition, the embodiment has a single orifice in the form of a three-way connector 62, that separates input channel 60 and output channel 61. Like Embodiment 3, the compliance mechanism further comprises a second fan 116 provided in the output channel 61 aimed at providing a pressure difference over the second fan. This second fan powers the exsufflation phase and thus reduces the impedance encountered by the return gas flow. Likewise inflow and outflow connections are attached near the body cavity access. In the outlet channel e.g. smoke can be filtered by a gas filter 63 for filtering smoke particles.

Embodiment 5 shown in Figure 3B combines the specific advantages of the Embodiments 1 and 2 in order to warrant high performances and smaller overall dimensions of the device. In this embodiment an insufflation gas reservoir made of a motorised piston-cylinder system 113, similar in function but smaller in volumes compared to one in Embodiment 2, is used to allow fast control actions on the pressure inside the overall system. This fan 112 will manage fast pressure transients that are limited in the total amount of volume changes by the smaller dimension of the reservoir. Servo-controlled fan 112 therefore used to manage faster frequencies and small amplitude pressure changes. In this embodiment the fast pressure changes are therefore first compensated by the fan 112, followed by the change in pressure provided by the slower dynamic of the piston 115, allowing combining the very fast reaction time of the fan to the slower but larger and less load-independent variation of pressure provided by the piston 115, resulting in a more performant and smaller device.

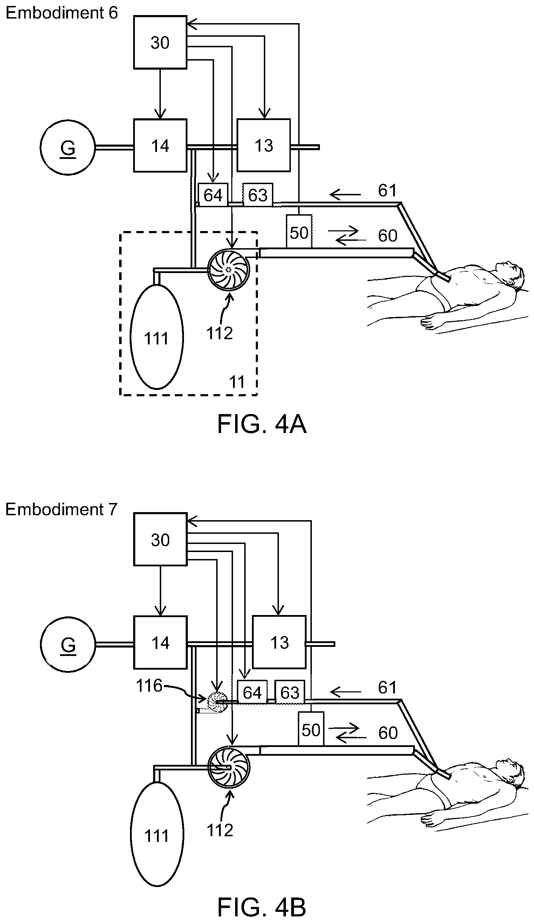

Figure 4A shows an embodiment wherein the pressure source can be any of those previously described, although preferably is a fan 112. The pressure source draws gas from a gas source G, which in the case of a turbine creates a constantly lower pressure in the reservoir than in the insufflated body cavity. A gas return channel 61 allows a small flow of insufflation gas back into the reservoir 111, creating a continuous or intermittent circulating gas flow. This gas return channel 61 can be provided with a gas filter 63 for filtering out smoke, fluids or other gas contamination. The gas return line 61 can be connected either to a different trocar, so that the output channel and the input channel originate from distinct orifices connected to the body cavity or to a three-way connector 62 placed at the connection between the trocar and the insufflation line 60. Using a single orifice, connections with the patient are simplified and the two lines (insufflation and gas return) can be also embedded in a single double-lumen tube. It is noteworthy that the three-way connector configuration can be effective only in combination with the low-impedance insufflator: the constant pressure concept of this invention also leads to a pendulum gas motion inside-outside the cavity through the trocar and the three-way connector. In the case of the presence of the gas return channel, the gas transitorily exiting the body cavity trough the trocar due to an inspiration of the patient or to any other reason will be drawn into the gas return channel and replaced by fresh gas entering from the insufflation line. This creates a cavity washout gas flow which can be used for multiple purposes. By providing a filter 63 smoke, fluids or other contamination can be removed from the insufflation gas. In addition, the circulating gas flow allows humidification and/or heating by providing the means to do so within the circuit or insufflation device. The gas-return line can be also provided by a return gas regulator valve 64 in the output channel. The valve 64 sets a relative flow resistance to the input line 60 to regulate return gas flow.

Embodiment 7, depicted in Figure 4B shows an additional second fan or compressor 116 provided in the return gas flow channel 61 which functions as a leak channel next to the insufflation input channel, as in embodiment 5. The impedance of this leak channel can be actively reduced by the second ventilator 116 in order to generate an increase in circulating flow. The advantage is a more efficient ability to filter smoke and apply humidification and heating.

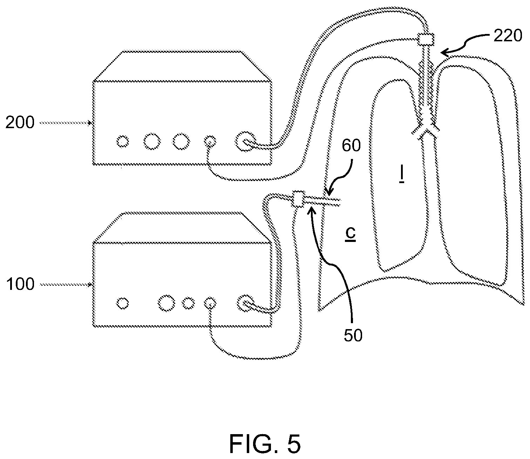

Figure 5 generically shows the function of the insufflator 100 having input mechanism 60. Typically, for minimally invasive surgery, the surgical instruments and an endoscopic camera are inserted into the thoracic or abdominal cavity or through trocars, that may simultaneously function as insufflator input mechanism 60. In some instance, the insufflator input mechanism 60 may be a trocar, that can be sealingly inserted in the cavity. In some embodiments, the trocar may have a venting mechanism that vents the insufflator output from the body cavity. Such an embodiment can be used when it is desired to (additionally) control the gas flow near the input mechanism of the insufflator. Such a venting mechanism may be passive, but the venting mechanism may also be actively operated by the insufflator controller. Additionally, the trocar can be used for insertion of a camera. In addition a breathing controller 200 with an intubation tube 220 may be provided that is typically used in conjunction with the insufflator, when a patient is in surgery. For this purpose they are equipped with sensors and the measurements are sent back insufflator 100. A pressure and/or flow sensor 50 is arranged in or near the body cavity for measuring a pressure level in the body cavity. To this end, in the example, sensor 50 is connected to the body cavity and provides real time data comprising at least comprising of at least one of an insufflation flow, insufflation output volume and insufflation pressure values for feedback to the insufflator 100.

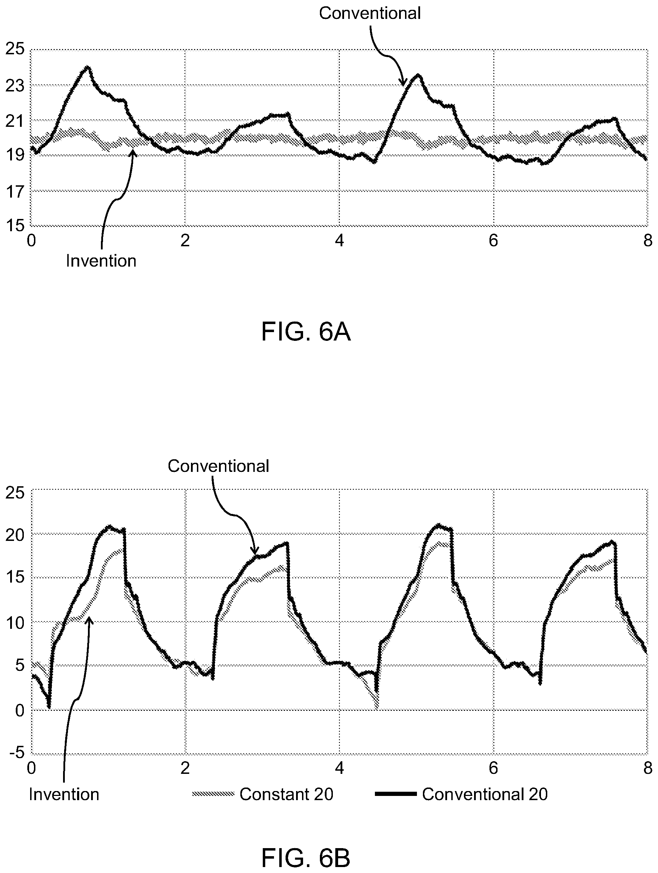

Figure 6A shows a comparison for an insufflator system held at 20 hPa, in actual operating conditions derived from in vivo experiments in a porcine model. It can be seen that the conventional pressures varies with peaks higher than 23 hPa, and below 19.5 hPa. In contrast, the fan-based insufflator with a gas reservoir (Embodiment 1), with a Douglas bag as a high compliance reservoir, pressure sensors, a control unit and a fan for keeping the pressure constant, was able to keep the pressure more constant. The results are significantly better, with a pressure variation kept between 2 hPa, with pieks no higher than 20.5 hPa.

Similarly, in Figure 6B it is shown how the breathing controller reacts to the enhanced insufflator. Because the abdomen is now kept almost at a virtual constant level, pressure peaks in the mechanical ventilation are also significantly lower, e.g. with peaks 2 or 3 hPa lower than the conventional setup. This evidently exposed to an overall lower pressure, while the possibilities for performing operations are improved.

### ADVANTAGE

The present invention may have following advantages. This invention entails a system that allows gas flowing in and out of the body cavity through a pressure generator that has an effective low impedance, allowing for automatic fast compensation for changes in the cavity pressure that are caused by whichever events such as mechanical ventilation, coughing , etc, while maintaining a constant insufflation pressure. The unique point of this technology is its fast adaptation to any changes in pressure by allowing a low impedance two-way gas flow to maintain the cavity pressure constant (and not allowing deflation only as consequence of overpressure) by limiting the release of insufflation gas. There are several other advantages:
- a reduction in the required ventilation pressures during insufflation.
- blood pressures are less opposed by insufflation pressures.
- coughing does not lead to harmful increases in insufflation and ventilation pressure
- no need of neither device synchronisation-communication with mechanical ventilators nor external sensor for probing ventilation pressures.
- When using a secondary return line this will create a circulating gas flow due to ventilation-induced pressure changes and consequential flow. This gas flow permits constant filtering of gas (e.g. smoke), and efficient humidification and heating.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. The mere fact that certain measures are recited in mutually different claims does not indicate that a combination of these measures cannot be used to advantage. As described above, the exemplary embodiments can be in the form of computer-implemented processes and apparatuses for practicing those processes. The exemplary embodiments can also be in the form of computer program code containing instructions embodied in tangible media, such as floppy disks, CD ROMs, hard drives, or any other computer-readable storage medium, wherein, when the computer program code is loaded into and executed by a computer, the computer becomes an apparatus for practicing the exemplary embodiments. The exemplary embodiments can also be in the form of computer program code, for example, whether stored in a storage medium, loaded into and/or executed by a computer, or transmitted over some transmission medium, loaded into and/or executed by a computer, or transmitted over some transmission medium, such as over electrical wiring or cabling, through fiber optics, or via electromagnetic radiation, wherein, when the computer program code is loaded into an executed by a computer, the computer becomes an apparatus for practicing the exemplary embodiments. When implemented on a general-purpose microprocessor, the computer program code segments configure the microprocessor to create specific logic circuits.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments disclosed for carrying out this invention, but that the invention will include all embodiments falling within the scope of the claims. Moreover, the use of the terms first, second, etc. do not denote any order or importance, but rather the terms first, second, etc. are used to distinguish one element from another. Furthermore, the use of the terms a, an, etc. do not denote a limitation of quantity, but rather denote the presence of at least one of the referenced item.

## Claims

1. An insufflator apparatus for exposing structures within a cavity of the human body for a diagnostic and/or therapeutic endoscopic procedure, comprising:
- an insufflation gas supply valve, adapted to provide insufflation gas to a pressure regulator;
- the pressure regulator, adapted to supply insufflation gas into the cavity of the human body via an input mechanism attachable to the human body;
- a means for determining a pressure level in the body cavity;
- an insufflator vent mechanism adapted to release excess insufflation gas volume returning from the pressure regulator;
- an insufflator controller arranged to real-time adapt an insufflation rate of said insufflator gas via said gas supply valve and vent mechanism at a set average pressure level in the body cavity in accordance with the means for determining the pressure level in the body cavity;
- wherein the pressure regulator has a limited volume for temporarily buffering a gas returning from the body cavity to thereby avoid transient pressure deviations from the set average pressure level in the body cavity, e.g. due to coughing or mechanical ventilation and allowing the buffered gas to return to the body cavity to maintain the set average pressure.

2. An insufflator apparatus according to claim 1, wherein the pressure regulator comprises a variable volume reservoir, e.g. a bag or bellow, communicatively coupled to the input channel.

3. An insufflator apparatus according to any preceding claim, wherein the pressure regulator comprises a piston-controlled cylinder communicatively coupled to the input channel, wherein the piston is actively controlled by the insufflator controller in accordance with the means for determining the pressure level in the body cavity.

4. An insufflator apparatus according to any preceding claim, wherein the pressure regulator comprises a fan provided in the input channel controlled by the insufflator controller in accordance with the means for determining the pressure level in the body cavity, to provide a controllable pressure difference over the fan to thereby counteract pressure variations deviating from the set average pressure level.

5. An insufflator apparatus according to any preceding claim, wherein the return gas flow is provided via an output channel for providing a return gas flow from the body cavity via a channel at least partially distinct from the input channel.

6. An insufflator apparatus according to claim 5, wherein the output channel and the input channel originate from a single orifice connected to the body cavity.

7. An insufflator apparatus according to claim 5, wherein the output channel and the input channel originate from distinct orifices connected to the body cavity.

8. An insufflator apparatus according to any of claims 5 -7, wherein the pressure regulator further comprises an additional fan or compressor provided in the output channel aimed at providing a pressure difference across the output channel to promote gas circulation.

9. An insufflator apparatus according to any of claims 5 - 8, wherein a gas filter is provided in at least one of the channels.

10. An insufflator apparatus according to any of claims 5 - 9, wherein a return gas regulator valve is provided in the output channel.

11. An insufflator apparatus according to any previous claim wherein the input channel comprises a humidifier arranged to humidify input insufflation gas.

12. An insufflator apparatus according to any of the preceding claims, wherein the insufflator controller is arranged to real-time adapt at least one of a gas flow, gas output volume and gas output pressure of the insufflator.

13. An insufflator apparatus according to any of the preceding claims, wherein the insufflator comprises a detector communicatively coupled to the insufflator controller, arranged to measure at least one of a gas flow, gas output volume and gas output pressure at the insufflator gas output.

14. An insufflator apparatus according to any of the preceding claims, wherein the insufflator comprises a detector arranged to measure at least one of a gas flow, gas output volume and gas output pressure and gas temperature at a distal part of the insufflator input mechanism.

15. An insufflator apparatus according to any of the preceding claims, wherein the insufflator input mechanism is a trocar, that can be sealingly inserted in the cavity.

16. A method of operating an insufflator comprising an insufflator gas supply, wherein the insufflator gas supply comprises a pressure regulator, having a limited volume for temporarily buffering a gas returning from a cavity to thereby avoid transient pressure deviations from the set average pressure level in the cavity, and allowing the gas to return to the cavity to maintain the set average pressure, comprising the steps of sensing a pressure by a pressure detector within the inssufflator; controlling the pressure within the cavity based on the sensed pressure of the pressure detector, by real-time adapting an insufflation rate of said insufflator gas at a set average pressure level in accordance with the sensed pressure; and controlling pressure and/or volume of the limited buffering volume.

17. A computer program product comprising program code stored on a machine-readable medium, the program code being configured, when executed by a processor, to carry out the method according to claim 16.

## Patentansprüche

1. Eine Insufflationsapparat zum Freilegen von Strukturen innerhalb einer Körperhöhle des menschlichen Körpers für ein diagnostisches und/oder therapeutisches endoskopisches Verfahren, umfassend:
- ein Insufflationsgaszufuhrventil, das dazu ausgelegt ist, einem Druckregler Insufflationsgas bereitzustellen;
- den Druckregler, der dazu ausgelegt ist, über einen am menschlichen Körper anbringbaren Eingabemechanismus Insufflationsgas in die Körperhöhle des menschlichen Körpers zuzuführen;
- ein Mittel zum Bestimmen eines Druckniveaus in der Körperhöhle;
- einen Insufflationsentlüftungsmechanismus, der dazu ausgelegt ist, überschüssiges, von dem Druckregler zurückströmendes Insufflationsgasvolumen abzulassen;
- eine Insufflationssteuerung, die dazu eingerichtet ist, eine Insufflationsrate des Insufflationsgases über das Gaszufuhrventil und den Entlüftungsmechanismus in Echtzeit bei einem eingestellten durchschnittlichen Druckniveau in der Körperhöhle gemäß dem Mittel zum Bestimmen des Druckniveaus in der Körperhöhle anzupassen;
- wobei der Druckregler ein begrenztes Volumen zum vorübergehenden Puffern eines aus der Körperhöhle zurückströmenden Gases aufweist, um dadurch vorübergehende Druckabweichungen von dem eingestellten durchschnittlichen Druckniveau in der Körperhöhle, z. B. aufgrund von Husten oder mechanischer Beatmung, zu vermeiden und dem gepufferten Gas zu ermöglichen, in die Körperhöhle zurückzuströmen, um den eingestellten durchschnittlichen Druck aufrechtzuerhalten.

2. Insufflationsapparat nach Anspruch 1, wobei der Druckregler einen Reservoir mit variablem Volumen, z. B. einen Beutel oder einen Balg, umfasst, das kommunikativ mit dem Eingangskanal gekoppelt ist.

3. Insufflationsapparat nach einem der vorhergehenden Ansprüche, wobei der Druckregler einen kolbengesteuerten Zylinder umfasst, der kommunikativ mit dem Eingangskanal gekoppelt ist, wobei der Kolben durch die Insufflationssteuerung gemäß dem Mittel zum Bestimmen des Druckniveaus in der Körperhöhle aktiv gesteuert wird.

4. Insufflationsapparat nach einem der vorhergehenden Ansprüche, wobei der Druckregler einen in dem Eingangskanal vorgesehenen Ventilator umfasst, der durch die Insufflationssteuerung gemäß dem Mittel zum Bestimmen des Druckniveaus in der Körperhöhle gesteuert wird, um eine steuerbare Druckdifferenz über den Ventilator bereitzustellen und dadurch Druckschwankungen entgegenzuwirken, die von dem eingestellten durchschnittlichen Druckniveau abweichen.

5. Insufflationsapparat nach einem der vorhergehenden Ansprüche, wobei der Rückgasstrom über einen Ausgangskanal zum Bereitstellen eines Rückgasstroms aus der Körperhöhle über einen zumindest teilweise von dem Eingangskanal getrennten Kanal bereitgestellt wird.

6. Insufflationsapparat nach Anspruch 5, wobei der Ausgangskanal und der Eingangskanal von einer einzigen mit der Körperhöhle verbundenen Öffnung ausgehen.

7. Insufflationsapparat nach Anspruch 5, wobei der Ausgangskanal und der Eingangskanal von getrennten, mit der Körperhöhle verbundenen Öffnungen ausgehen.

8. Insufflationsapparat nach einem der Ansprüche 5-7, wobei der Druckregler ferner einen zusätzlichen Ventilator oder Kompressor umfasst, der in dem Ausgangskanal vorgesehen und dazu bestimmt ist, eine Druckdifferenz über den Ausgangskanal bereitzustellen, um die Gaszirkulation zu fördern.

9. Insufflationsapparat nach einem der Ansprüche 5-8, wobei in mindestens einem der Kanäle ein Gasfilter vorgesehen ist.

10. Insufflationsapparat nach einem der Ansprüche 5-9, wobei in dem Ausgangskanal ein Rückgasregelventil vorgesehen ist.

11. Insufflationsapparat nach einem der vorhergehenden Ansprüche, wobei der Eingangskanal einen Befeuchter umfasst, der dazu eingerichtet ist, das zugeführte Insufflationsgas zu befeuchten.

12. Insufflationsapparat nach einem der vorhergehenden Ansprüche, wobei die Insufflationssteuerung dazu eingerichtet ist, mindestens eines von einem Gasstrom, einem Gasausgangsvolumen und einem Gasausgangsdruck des Insufflationsapparats in Echtzeit anzupassen.

13. Insufflationsapparat nach einem der vorhergehenden Ansprüche, wobei der Insufflationsapparat einen kommunikativ mit der Insufflationssteuerung gekoppelten Detektor umfasst, der dazu eingerichtet ist, mindestens eines von einem Gasstrom, einem Gasausgangsvolumen und einem Gasausgangsdruck an dem Insufflationsgasausgang zu messen.

14. Insufflationsapparat nach einem der vorhergehenden Ansprüche, wobei der Insufflationsapparat einen Detektor umfasst, der dazu eingerichtet ist, mindestens eines von einem Gasstrom, einem Gasausgangsvolumen und einem Gasausgangsdruck sowie eine Gastemperatur an einem distalen Teil des Insufflationseingabemechanismus zu messen.

15. Insufflationsapparat nach einem der vorhergehenden Ansprüche, wobei der Insufflationseingabemechanismus ein Trokar ist, der abdichtend in die Körperhöhle eingeführt werden kann.

16. Verfahren zum Betreiben eines Insufflationsapparats, der eine Insufflationsgaszufuhr umfasst, wobei die Insufflationsgaszufuhr einen Druckregler umfasst, der ein begrenztes Volumen zum vorübergehenden Puffern eines aus einer Körperhöhle zurückströmenden Gases aufweist, um dadurch vorübergehende Druckabweichungen von dem eingestellten durchschnittlichen Druckniveau in der Körperhöhle zu vermeiden und dem Gas zu ermöglichen, in die Körperhöhle zurückzuströmen, um den eingestellten durchschnittlichen Druck aufrechtzuerhalten, umfassend die Schritte des Erfassens eines Drucks durch einen Druckdetektor innerhalb des Insufflationsapparats; des Steuerns des Drucks innerhalb der Körperhöhle auf Grundlage des durch den Druckdetektor erfassten Drucks durch Anpassen einer Insufflationsrate des Insufflationsgases in Echtzeit bei einem eingestellten durchschnittlichen Druckniveau gemäß dem erfassten Druck; und des Steuerns des Drucks und/oder Volumens des begrenzten Puffervolumens.

17. Computerprogrammprodukt, umfassend einen auf einem maschinenlesbaren Medium gespeicherten Programmcode, wobei der Programmcode dazu konfiguriert ist, bei Ausführung durch einen Prozessor das Verfahren nach Anspruch 16 durchzuführen.

## Revendications

1. Appareil insufflateur pour exposer des structures à l'intérieur d'une cavité du corps humain pour une procédure de diagnostic et/ou endoscopique thérapeutique, comprenant :
une valve d'alimentation en gaz d'insufflation, adaptée pour fournir le gaz d'insufflation à un régulateur de pression ;
le régulateur de pression, adapté pour fournir le gaz d'insufflation dans la cavité du corps humain via un mécanisme d'entrée pouvant être fixé au corps humain ;
un moyen pour déterminer un niveau de pression dans la cavité corporelle ;
un mécanisme de mise à l'évent d'insufflateur adapté pour libérer le volume de gaz d'insufflation en excès revenant du régulateur de pression ;
un organe de commande d'insufflateur agencé pour adapter, en temps réel, un débit d'insufflation dudit gaz d'insufflation via ladite valve d'alimentation en gaz et le mécanisme de mise à l'évent à un niveau de pression moyenne de consigne dans la cavité corporelle selon le moyen pour déterminer le niveau de pression dans la cavité corporelle ;
dans lequel le régulateur de pression présente un volume limité destiné à recevoir temporairement le gaz revenant de la cavité corporelle afin d'éviter ainsi des écarts transitoires du niveau de pression moyenne de consigne dans la cavité corporelle, par exemple dus au fait de tousser ou à la ventilation mécanique et permettre au gaz reçu de retourner dans la cavité corporelle afin de maintenir la pression moyenne de consigne.

2. Appareil d'insufflateur selon la revendication 1, dans lequel le régulateur de pression comprend un réservoir à volume variable, par exemple un sac ou un soufflet, couplé, par communication, au canal d'entrée.

3. Appareil d'insufflateur selon l'une quelconque des revendications précédentes, dans lequel le régulateur de pression comprend un cylindre commandé par piston couplé, par communication, au canal d'entrée, dans lequel le piston est activement commandé par l'organe de commande d'insufflateur selon le moyen pour déterminer le niveau de pression dans la cavité corporelle.

4. Appareil d'insufflateur selon l'une quelconque des revendications précédentes, dans lequel le régulateur de pression comprend un ventilateur prévu dans le canal d'entrée commandé par l'organe de commande d'insufflateur selon le moyen pour déterminer le niveau de pression dans la cavité corporelle, afin de fournir une différence de pression contrôlable de part et d'autre du ventilateur afin de contrecarrer ainsi les variations de pression par rapport au niveau de pression moyenne de consigne.

5. Appareil d'insufflateur selon l'une quelconque des revendications précédentes, dans lequel le flux de gaz de retour est fourni, via un canal de sortie, pour fournir un flux de gaz de retour de la cavité corporelle via un canal au moins partiellement distinct du canal d'entrée.

6. Appareil d'insufflateur selon la revendication 5, dans lequel le canal de sortie et le canal d'entrée prennent naissance au niveau d'un seul orifice raccordé à la cavité corporelle.

7. Appareil d'insufflateur selon la revendication 5, dans lequel le canal de sortie et le canal d'entrée prennent naissance au niveau d'orifices distincts raccordés à la cavité corporelle.

8. Appareil d'insufflateur selon l'une quelconque des revendications 5 à 7, dans lequel le régulateur de pression comprend en outre un ventilateur supplémentaire ou compresseur prévu dans le canal de sortie ayant pour but de fournir une différence de pression sur le canal de sortie afin de favoriser la circulation de gaz.

9. Appareil d'insufflateur selon l'une quelconque des revendications 5 à 8, dans lequel un filtre à gaz est prévu dans au moins l'un des canaux.

10. Appareil d'insufflateur selon l'une quelconque des revendications 5 à 9, dans lequel une valve de régulateur de gaz de retour est prévue dans le canal de sortie.

11. Appareil d'insufflateur selon l'une quelconque des revendications précédentes, dans lequel le canal d'entrée comprend un humidificateur agencé pour humidifier le gaz d'insufflation d'entrée.

12. Appareil d'insufflateur selon l'une quelconque des revendications précédentes, dans lequel l'organe de commande d'insufflateur est agencé pour adapter, en temps réel, au moins l'un parmi un flux de gaz, un volume de sortie de gaz et une pression de sortie de gaz de l'insufflateur.

13. Appareil d'insufflateur selon l'une quelconque des revendications précédentes, dans lequel l'insufflateur comprend un détecteur couplé, en communication, à l'organe de commande d'insufflateur, agencé pour mesurer au moins l'un parmi un flux de gaz, un volume de sortie de gaz et une pression de sortie de gaz au niveau de la sortie de gaz d'insufflation.

14. Appareil d'insufflateur selon l'une quelconque des revendications précédentes, dans lequel l'insufflateur comprend un détecteur agencé pour mesurer au moins l'un parmi un flux de gaz, un volume de sortie de gaz et une pression de sortie de gaz et la température de gaz au niveau d'une partie distale du mécanisme d'entrée d'insufflateur.

15. Appareil d'insufflateur selon l'une quelconque des revendications précédentes, dans lequel le mécanisme d'entrée d'insufflateur est un trocart, qui peut être inséré, de manière étanche, dans la cavité.

16. Procédé pour actionner un insufflateur comprenant une alimentation en gaz d'insufflation, dans lequel l'alimentation en gaz d'insufflation comprend un régulateur de pression, ayant un volume limité pour tamponner temporairement un gaz revenant d'une cavité afin d'éviter ainsi les écarts de pression transitoires par rapport au niveau de pression moyenne de consigne dans la cavité, et permettant au gaz de retourner dans la cavité afin de maintenir la pression moyenne de consigne, comprenant les étapes comprenant le fait de détecter une pression par un détecteur de pression à l'intérieur de l'insufflateur ; le fait de contrôler la pression à l'intérieur de la cavité sur la base de la pression détectée du détecteur de pression, en adaptant en temps réel un débit d'insufflation dudit gaz d'insufflation à un niveau de pression moyenne de consigne selon la pression détectée ; et le fait de contrôler la pression et/ou le volume du volume tampon limité.

17. Produit de programme informatique comprenant un code de programme stocké sur un support lisible par machine, le code de programme étant configuré, lorsqu'il est exécuté par un processeur, pour réaliser le procédé selon la revendication 16.
